# EUROPÄISCHE PATENTANMELDUNG

(11) **EP 4 108 264 A1**
(43) Veröffentlichungstag der Anmeldung: **28.12.2022**
(21) Anmeldenummer: 22000155.6
(22) Anmeldetag: 14.06.2022
(51) Int. Cl.: A61L 2/28, A61L 2/07

(54) **STERILISATIONSVORRICHTUNG MIT EINER KONTROLLVORRICHTUNG**

(30) Priorität: 21.06.2021 DE 102021003190
(71) Anmelder: Simmoteit, Robert, 72414 Rangendingen (DE)
(72) Erfinder: Simmoteit, Robert, 72414 Rangendingen (DE)

(57) **Zusammenfassung**

Sterilisationsvorrichtung für Sterilisationsprodukte, die in Sterilisationsgeräten zum Einsatz kommt, wobei die Sterilisationsprodukte in einem Sterilbehälter oder einer transparenten Weichverpackung als Sterilisationsverpackung eingebracht sind, dadurch gekennzeichnet, dass eine Kontrollvorrichtung (6, 23) zumindest eine Kontrollfläche (18, 31) im inneren einer Sterilisationsverpackung so anordnet, dass das Sterilisationsmedium bei der Sterilisation zumindest die Kontrollfläche in der Kontrollvorrichtung über eine offene Seite (26), eine Öffnung oder einen Kanal (8) und/oder über ein Hindernis (25, 31) erreicht, um eine sichtbare Flächenänderung der Kontrollfläche zu bewirken und diese ohne Öffnen der Sterilisationsverpackung zumindest außerhalb des Sterilisationsgerätes visuell oder mit optischen Methoden auszulesen oder zu analysieren.

## Beschreibung

### Gebiet der Erfindung

Sterilisationsvorrichtung für Sterilisationsprodukte mit einer Kontrollvorrichtung zur Prüfung der Sterilisation im Bereich der medizinischen Produktaufbereitung.

### Hintergrund der Erfindung

Kontaminierte medizinische Instrumente werden in der Regel in einem medizinischen Aufbereitungsprozess (Reinigung, Sterilisierung, Verpackung und Transport) für den nächsten medizinischen Einsatz wiederhergestellt. Um die Instrumente sterilisieren zu können, werden Sterilisationsgeräte und Sterilisationsbehälter eingesetzt.

Sterilbehälter nehmen insbesondere eine Siebschale oder ein Siebtray mit medizinischen Instrumenten und Geräten wie Kanülen, Endoskope, Motoren, Bohrern oder ähnliche Instrumente auf. Diese Vorrichtungen werden mit einem Sterilbehälterdeckel verschlossen und in Sterilisierungsgeräte verbracht, in denen die medizinischen Instrumente und Geräte nach einem validierten Verfahren sterilisiert werden. Um zu erkennen, ob eine Sterilisation erfolgt ist, werden Sterilisationsettiketten mit einem temperatursensitiven Farbindikator außen an einer Halterung eingesetzt.

In Weiterbildung DE 10 2017 117 624 A1 wird ein modular aufgebauter Deckel für einen Sterilbehälter beschrieben der einen perforierten Gasaustauschabschnitt mit zumindest einer teilweise transparenten Gitterabdeckung für einen Sterilfilter aufweist und diese Abdeckung abnehmbar ist. Zum Stand der Technik gehören weiter Sterilbehälter mit einem außen angebrachten Verschlusssystem, an dem Sterilblomben oder Sterilettiketten anbringbar sind.

Derzeit ist nicht sichergestellt, dass der durchgeführte Sterilisationsprozess von Sterilisationsbehältern im Inneren korrekt abgelaufen ist und damit sichergestellt wird, dass dieser zu 100% sterile medizinische Geräte und Instrumente enthält, denn der Sterilisationserfolg wird nicht im inneren des Sterilcontainers überprüft. Die äußere Kontrolle ist darüber hinaus eher ein Prozesshinweis, dass eine Sterilisation stattgefunden hat.

### Zusammenfassung der Erfindung

Die Neuerung besteht vorzugsweise aus einem neuartigen Deckel, der als wesentlicher Bauteil an einem Sterilisationsbehälter zum Einsatz kommt, wobei der Deckel ein wannenartiges Behälterteil verschließt. Dabei ist angedacht, die Neuerung auch im wannenartigen Behälterteil zu nutzen, also dieser zumindest an den Seitenwänden des Behälters zum Einsatz kommt. Dabei muss die Neuerung sich im Inneren des Sterilisationsbehälterraumes befinden und von Sterilisationsmedien erreichbar oder zumindest oberflächlich anströmbar sein. Desweiteren ist die Neuerung so aufgebaut, dass zumindest eine visuelle Sichtkontrolle oder eine Bilddatenanalyse mit Geräten (Kamera, Scanner oder mit ähnlichen Geräten erfolgen kann) und/oder eine andersartige Datenübermittlung ermöglicht wird, um die durchgeführte Sterilisation im Inneren des Sterilcontainers bewerten zu können, ohne diesen öffnen zu müssen. Denkbar sind aber auch mechanische Vorrichtungen, die über den Deckel in den Sterilbehälter eingebracht werden.

Als weitere Aufgabe ist angedacht, die Neuerung zur Validierung von medizinischen Aufbereitungsprozessen einzusetzen oder zumindest den Sterilisationsprozess im Behälterinneren ohne technisches Zubehör überprüfbar zu machen. Wesentlich ist dabei, dass die enthaltenden medizinischen Geräte und Instrumente auch wirklich in der Sterilverpackung sterilisiert wurden und nicht nur die äußere Sterilisationshülle des Sterilisationsbehälters oder die einer Weichverpackung.

Die Aufgabe wird erfindungsgemäß durch eine Sterilisationsvorrichtung für Medizinprodukte zur Prüfung der Sterilisation im Bereich der medizinischen Produktaufbereitung nach Anspruch 1 gelöst, wobei eine Kontrollvorrichtung nach Anspruch 9 mit einer Kontrollfläche nach Anspruch 11 eingesetzt ist. Die folgenden oder abhängigen Ansprüche betreffen die vorteilhaften Ausgestaltungen der Erfindung.

Die Neuerung löst die Aufgabe durch eine Sterilisationsvorrichtung mit einer Kontrollvorrichtung für Sterilisationsprodukte, die in Sterilisationsgeräten zum Einsatz kommt, wobei die Sterilisationsprodukte in einem Sterilbehälter oder einer transparenten Weichverpackung als Sterilisationsverpackung eingebracht sind, dadurch gekennzeichnet, dass eine Kontrollvorrichtung zumindest eine Kontrollfläche im inneren einer Sterilisationsverpackung so anordnet, dass das Sterilisationsmedium bei der Sterilisation zumindest die Kontrollfläche in der Kontrollvorrichtung über eine offene Seite, eine Öffnung oder einen Kanal und/oder über ein Hindernis erreicht, um eine sichtbare Flächenänderung der Kontrollfläche zu bewirken und diese ohne Öffnen der Sterilisationsverpackung die Flächenänderung zumindest außerhalb des Sterilisationsgerätes visuell zugänglich ist oder mit optischen Methoden auszulesen oder zu analysieren.

Die Kontrollvorrichtung kann dabei ein fester Bestandteil eines Sterilbehälterdeckels oder eine auswechselbare oder einlegbare Einheit sein die über ein transparentes Fenster oder Folie einer Weichverpackung optisch erfassbar ist oder eine Lupe, einen Lichtleiter und/oder ein Kameramodul besitzt, um zumindest eine visuelle oder optische Analyse der hierunterliegenden oder zugänglichen, vorzugsweise einer beabstandeten Kontrollfläche zu ermöglichen. Es kann dabei vorgesehen sein, dass eine optische Analyse bereits im Sterilisationsgerät über ein Kabel oder über Funkt (Bluetooth) erfolgt. Weiter kann vorgesehen sein, dass die Kontrollvorrichtung oder nur die Kontrollfläche erste bei der Reinigung und anschließend bei der Sterilisation eingesetzt wird oder diese bereits Bestandteil eines Siebkorbs ist und als Lagersystem eingebracht wird.

Die wesentliche Neuerung berücksichtigt hierbei die real verwendeten Sterilisationsbarrieren (Sterilfilter, Weichverpackungen ect.) und ermöglicht ohne Öffnen der Sterilverpackung die Bewertung eines Sterilisationsprozesses z. B. über Kontrollflächen. Weiterer Vorteil ist, dass außerhalb der Sterilisationsverpackung die Visualisierung durch Hilfsmittel wie Luppen oder das Auslesen über Scanner oder visuell erfolgen kann. Damit wird die Genauigkeit und Qualität der Analyse nicht nur durch die Beschaffenheit der Kontrollfläche angezeigt, sondern durch deren Visualisierung welche auch über Geräte wie Scanner oder Kameras auch quantifizierbar sein kann. Ferner ermöglichen geeignete Sperrflächen oder Hindernisse eine zeitliche Verzögerung z. B. der Dampfeinwirkung, da das Sterilisationsmedium wie Dampf diese erst überwinden muss. Der Farbwechsel wird so zeitverzögernd angezeigt. Es findet zumindest eine zeitversetzte Einwirkzeit statt und/oder durch das Hindernis wird die Einwirkkonzentration des Sterilisationsmediums reduziert. Letzteres kann zur Simulation eines "worst case" Falles herangezogen werden oder ist für eine Sicherheitsaussage zur erfolgten Sterilisation von Bedeutung. Wesentlich in der vorgeschlagenen Neuerung ist, dass die vorgeschlagene Kontrollvorrichtung in einem Sterilisationsprozess ähnlich behandelt wird und das zu sterilisierende Produkt und sich dabei in einer Sterilisationsverpackung befindet.

Die hier eingebrachten Sterilisationsprodukte z. B. in Siebkörben können Medizinprodukte oder alternativ Testprodukte sein, um den Sterilisationserfolg hiermit bestimmen zu können. Insbesondere deren Ausgestaltung, deren Materialbeschaffung und Aufbau können für die Bewertung des Sterilisationserfolges herangezogen werden. Dabei kann vorgesehen sein, dass die Kontrollvorrichtung als eigenständige Vorrichtung in Verbindung mit einer Sterilverpackung (Hart- oder Weichverpckung) eingesetzt ist, um an einer oder an mehreren Stellen einer Sterilisationskammer den Sterilisationserfolg zu ermitteln oder die Kontrollvorrichtung aus einer Kontrollhalterung im Behälterinnraum angeordnet ist und von außen über einen transparenten Deckel einsehbar ist.

Die Neuerung kann am Sterilisationsbehälterdeckel einen Adapter in Verbindung mit einem Lichtleiter aufweisen, wobei das Lichtleiterende z. B. über eine Kappe eine endständige und beabstandete Kontrollfläche aufweist, um hierüber die eingewirkte Temperatur über Farbindikatoren zu visualisieren.

In einer weiteren Ausgestaltung kann die Neuerung an der Unterseite des Sterilbehälterdeckels einen Batteriebetriebenen Datenspeicher aufweisen, der in Verbindung mit einem Temperatursensor den Sterilisationsprozess Zeitaufgelöst aufzeichnet. Das Auslesen der Daten kann dabei kabellos oder über einen Anschlussadapter erfolgen.

Im Wesentlichem muss die Farbänderung eines Temperaturkontrollstreifens visuell oder mit einer Kamera oder mit einem Scanner ermittelbar sein oder die gespeicherten Daten über einen elektrischen Anschlussadapter oder eine Induktionskopplerfläche zur induktiven Datenübertragung vorhanden sein. Je nach Bauart kann aber auch die Datenermittlung über den Raum hinweg und durch dünne Materialschichten erfolgen. Für eine sichere Datenauslesung sollte ein möglichst direkter Kontakt mit der Induktionsfläche oder einem innenliegenden Datenspeicher möglich sein.

Die Neuerung sieht dabei eine Kontrolleinheit als integrierte Vorrichtung im Sterilbehälter vor oder die von außen in Form eines Siebkorbs oder als deren Bestandteils in diesen angebracht wird. Die Kontrolleinheit besitzt zumindest einen Kontrollhalter, der eine Kontrollfläche in Form eines Etiketts aufnimmt und hält oder einen Lichtleiter aufweist, der zumindest vorne eine Haltekappe mit einer Kontrollfläche aufnimmt oder an einer Kontrollfläche herangeführt ist, um diese bildlich zu erfassen. Die Kontrolleinheit oder zumindest der analytische Teil der Einheit muss während des Sterilisationsprozesses von den Sterilisationsmedien (z. B. Dampf) erreichbar sein. Von Vorteil ist, wenn dabei ein Teststreifen beabstandet und unter einer Deckelfläche angeordnet ist, die zumindest über einen Spalt oder eine Zugangsöffnung verfügt. Der eingesetzte Sterilbehälterdeckel kann vorzugsweise aus einem transparenten Kunststoff bestehen. Letzterer Deckel kann eine integrierte Kontrollstreifenhalterung aufweisen. Die Filterhalterung kann dabei eine transparente Filterschnecke sein oder aufnehmen, um hier eingebrachte Partikel und Fasern (Verunreinigungen) im Inneren der Filterschnecke beobachten und unter Kontrolle entfernen zu können. Transparente Filterschnecken haben dabei den weiteren Vorteil, dass hier entstehendes Kondenswasser beobachtbar ist. Denkbar sind somit transparente Filterschnecken oder Teile hiervon, um zumindest unter der Filterschecke eine Kontrollstreifenhalterung visuell sichtbar aufzunehmen.

Um die Farbänderung der Kontrollfläche nach dem Sterilisationsprozess visuell ohne Hilfsmittel zu beobachten oder auslesen zu können, befindet sich vorzugsweise eine transparente Fensterfläche in einer Sterilbehälterwandung bzw. in einem Sterilbehälterdeckel. Diese kann ein kleines Sichtfenster in Größe eines Teststreifens (z. B. 1 - 3 cm Breit und 3-10 lang) sein. Unter dieser Fensterfläche kann eine gut sichtbare und innenliegende Farbfläche beabstandet angeordnet sein. Von Vorteil ist, wenn die Farbfläche visuell oder mit einer Kamera oder Scanner auslesbar ist. Als geeignete Materialien sind transparente Temperaturstabile Folien oder Flächen z. B. aus Polytetrafluorethylen wie ePTFE oder aus Polyetheretherketon (Peek) denkbar. Technisch kann die Kontrolleinheit als transparentes Fenster oder als Luppe ausgeführt sein und eine darunterliegend beabstandete Halterung für einen Teststreifen aufweisen. Die Kontrolleinheit kann in einem Deckel eingearbeitet und dabei auswechselbar sein. Die Kontrolleinheit kann aber auch eine Vorrichtung sein, die einen Datenspeicher aufweist, der mit einem Temperatursensor verbunden ist oder im Inneren des Behälters eine Datenübermittlung vorsieht. Auch diese Vorrichtung sollte vorzugsweise austauschbar sein, um z. B. eine Batterie leichter auswechseln zu können.

Anstelle des Fensters oder einer Luppe kann ein Adapter vorhanden sein, der nach innen mit einem Lichtleiter verbunden ist, um hierüber Licht einzubringen, um damit die innenliegende Farbfläche auslesen zu können (z. B. vergleichbar mit Lichtleiter, die endoskopisch eingesetzt sind). Diese Vorrichtung kann dabei auch direkt in einem Instrumentenkanal eingebracht sein. Vorteil dieser Anordnung ist, dass eine Pixeländerung des Farbindikators erfassbar ist und damit elektronisch Daten miteinander verglichen werden können. Alternativ kann nach innen ein Temperatursensor vorhanden sein, der Temperaturdaten analysiert und diese an einem Datenspeicher weiterleitet. Die bevorzugte Kontrolleinrichtung ist dabei ein integrierter Bestandteil des Sterilisationsbehälters und keine selbständig mobil nutzbare Vorrichtung. Dabei ist vorgesehen, dass der Datenspeicher sich im Deckel oder an einer Seitenwandung der Behälterwanne befindet. Die Datenweiterleitung kann kabellos über Kontaktflächen (auch per Funk) oder über ein Kabel erfolgen. Geeignete Sensoren können an fast jedem Ort im Inneren des Sterilcontainers positioniert sein. Entsprechendes wird mit einem Lichtleiter oder Temperatursensor erreicht, der nahe am Sterilisationsgut oder sogar in diesem platzierbar ist, um hier die Temperatur im inneren Behälterraum ortsbezogen und/oder zeitaufgelöst zu erfassen.

Die Neuerung sieht zumindest eine visuell mit dem Auge erfassbare informative Kontrollfläche vor. Hierbei wird eine Farbänderung erfasst und zusätzlich eine Botschaft z. B. in Form eines Symbols oder eines Wortes, Textes ect., die erst nach dem Sterilisationsprozess sichtbar wird. Die Botschaft kann das Wort "Steril" oder "okay" oder ein Kontrollkästchen mit einem "X" oder "Hacken" sein. Denkbar sind auch Kombinationen, um den Betrachter eine eindeutige und unterstützende Information ohne Hilfsmittel zu vermitteln. Es versteht sich von selbst, dass derartige Teststreifen auch in anderen Einsatzgebieten wie die der Reinigung zur Anwendung kommen. Die Neuerung besitzt somit eine Kontrollfläche, dadurch gekennzeichnet, dass die Kontrollfläche eine Indikatorschicht und/oder eine Biomarkerschicht aufweist, wobei eine temperaturgetriebene chemische Farbänderung oder Abschmelzung einer oberen Schicht ein Symbol oder ein Symbol mit einem Zeichen oder Wort erzeugt oder freisetzt oder die abschmelzbare Schicht eine hierunter liegende Kontrollschicht mit hier enthaltenem Biomaterial freilegt. Dabei kann ein saugfähiges Material mit einem lösungsstabilen Trägermaterial verbunden sein, wobei die darüberliegende schmelzbare Sperr- oder Deckschicht durch die Biomarkerschicht diffundiert oder über Kanäle fließen kann oder diese bei einem Reinigungsprozess zuerst abgewaschen wird.

Denkbar ist aber auch ein neuer Ansatz einer Kontrollfläche eines Teststreifens oder Testvorrichtung, bei der über- oder nebeneinander einer Indikatorschicht oder in der Fläche eine Wachstumsschicht oder -fläche für Keime vorhanden ist. Diese Anordnung erlaubt die Visualisierung des Sterilisationserfolges und hiernach eine Sicherheitsprüfung mit der Wachstumsschicht für Keime, die sicherzustellt, dass keine Keime in der Sterilisationsverpackung (Sterilcontainer) mehr vorhanden und auch die Dichtungen oder Sperrschichten (Filter) des Sterilcontainers dicht sind oder Ihre Aufgabe erfüllen. Könnten Keime (Bakterien oder Pilze) in den sterilen Raum eindringen, würden diese eingedrungenen Keime auf der Wachstumsschicht (feste Nährböden) Kolonien bilden. Diese Neuerung ist dadurch gekennzeichnet, dass der Teststreifens bzw. die Kontrollfläche in der Sterilisationsvorrichtung eingebracht ist und als Kontrollfläche eine Wachstumsschicht für Keime aufweist, wobei diese über- oder nebeneinander einer Indikatorschicht angeordnet oder als eine Indikatorwachstumsschicht ausbildet ist. Vorzugsweise besteht die Wachtumschicht aus einer dünnen Agarschicht die bereits den Farbindikator enthält oder über einer Indikatorschicht angeordnet ist und bei der Sterilisation sich die Farbe der Indikatorschicht oder des Farbindikators ändert. Diese Ausgestaltung einer Kontrollfläche bietet damit optimale Vorrausetzungen zur Validierung von Sterilisationsverpackungen.

Von Vorteil ist, wenn die Kontrollfläche, die auch ein Label (bzw. Etikett) sein kann, eine Farbschicht und eine darüberliegende Sperrschicht (z. B. transparente Kunststoffschicht oder Wachsschicht, die zumindest im Dampf schmilzt) aufweist oder diese erst eine vorgeschaltete Hindernisstrecke überbrücken muss, um die Farbänderung zeitlich zu verzögern. Damit gibt nicht nur die Farbänderung nach der Sterilisation eine erreichte Temperatur an, sondern wird durch eine visuell erfassbare Botschaft verstärkt. Alternativ kann eine Deck- oder Sperrschicht, welche nicht transparent ist, durch Abschmelzen oder Abwaschen entfernt werden. Vorzugsweise wird nach der Entfernung der Sperr- oder Deckschicht hierunter eine andersfarbige Oberfläche, Markierung, Barcode oder Zeichen oder Wörter sichtbar. Diese wiederum gibt Informationen wieder, die nicht nur auf ein Temperaturereignis hindeuten, sondern erst dann erfolgen, wenn zuvor ein Ereignis stattgefunden hat oder dieses Ereignis die Vorrausetzung für ein weiteres Ereignis schafft.

Diese Art der Kontrollstreifen oder Etiketten bewerten ein Ereignis nach einem Zwei Phasen-Kontrollmodell. Beispielsweise wird in der ersten Phase z. B. über den Schmelzpunkt eine Sperr- oder Deckschicht entfernt und unter dieser Schicht eine zweite Schicht freigelegt, welche in einem weiteren Prozess abgereichert oder die hier vorhandenen Testkeime oder Infektionsmaterialen (Viren, Bakterien oder Sporen) abgetötet werden können. Derartige Phasen-Kontrollmodelle können bei der Sterilisierung und bei der Reinigung eine Rolle spielen. Derartige Kontrollstreifen bieten kostengünstige Lösungen zur ganzheitlichen Betrachtung von Sterilisations- und Reinigungsschritten. In Verbindung mit den obigen beschriebenen Kontrollvorrichtungen (z. B. in Ausgestaltung einer Kammer) wird eine sichere Prozessüberwachung erreicht, insbesondere weil die abgeschmolzenen Stoffe die zu sterilisierenden Produkte nicht kontaminieren dürfen.

Von Vorteil ist es, wenn die Farbflächen oder die freigelegten Flächen in welcher Form auch immer, als Pixel ausgelesen und erfasst werden. Erst so ist es möglich, diese über eine Kl einer Analysesoftware zu analysieren, um individuelle Fehler beim Auslesen und Bewerten zu minimieren oder zu verhindern. Letztere ist wiederum ein Qualitätskriterium einer eingebrachten Kontrollfläche oder eines Sterilisationsgerätes, wenn die Daten von vielen Nutzern gegenübergestellt werden.

Innovativ kann eine Indikatorfläche vorgesehen sein, welche über eine transparente Deckfläche verfügt, die die Funktion eines Temperaturisolators hat, um eine Zeitverzögerung der Temperatureinwirkung im Sterilisationsraum des Sterilbehälters zu erreichen. Dieser Temperaturisolator kann ein Bestandteil der Kontrollhalterung sein. Der Temperaturisolator kann dabei auch ein Gitter sein, um eine unterschiedliche Flächenwirkung zu erziehen. Also Flächen, die überdeckt sind und Flächen, die nicht überdeckt sind. Damit wird sichergestellt, dass die Temperatur nicht sofort eine chemische Reaktion auslöst, da es wichtig ist, dass die gesetzlichen Vorgaben wie eine Temperatur von 134 °C z. B. für eine Zeit von 5 min eingebacht wurden.

Es kann vorgesehen sein, dass ein Bestandteil der Kontrolleinheit einen Edelstahlbehälter mit z. B. einen Federelement ausweist, um den Teststreifen zu fixieren und dieser wiederum ein fester Bestandteil des Deckels ist. Ein Teil der Teststreifenhalterung kann dabei eine Ausformung in der Deckelfläche oder einer Siebkorbfläche sein, um eine Kontrolleinheit aufzunehmen oder selbst Bestandteil der Kontrolleinheit sein. Von Vorteil ist, wenn die Kontrollfläche soweit von der Deckelwandung angeordnet ist, damit der Dampf frei oder über zumindest einen Zuführkanal oder über einen seitlichen Öffnungsbereich einströmen kann, um eine hier vorhandene Kontrollfläche mit einem Temperaturindikator zu überströmen oder zu erreichen. Die Neuerung löst somit die Aufgabe mit einer Kontrollvorrichtung die zumindest aus einem Sichtfenster und hierunterliegend einen flächigen Teststreifenhalter besteht, der z. B. ein Federelement besitzt, um einen Teststreifen zu halten und diese Vorrichtung einen für das Sterilisationsmedium erreichbaren Kontrollraum aufweist und dabei diese Vorrichtung mit einem Sterilbehälterdeckel lösbar verschraubt oder in einer Weichverpackung eingebracht ist.

Im Falle von Weichverpackungen mit zumindest einer transparenten Folie, kann in dieser eine Kontrollvorrichtung einzeln oder in einem Siebkorb vorgesehen sein, die eine Kammer mit einem transparenten Fenster besitzt. In diese Kammer ist ein Kontrollstreifen eingebracht und an diesen befindet sich eine geeignete Öffnung oder zuführende Leitung, um hierüber das Sterilisationsmedium (Dampf, H2O2, ETO ect.) in die Kontrollkammer zu leiten. Auch in dieser Anordnung kann der Sterilisationserfolg im Inneren einer Weichverpackung bestimmt werden. Derartige Kontrollvorrichtungen können dabei auch unabhängig von einer Umverpackung eingesetzt sein, um den Sterilisationsprozess zu überwachen. Wesentlich ist dabei, dass der Eintritt des Sterilisationsmediums durch ein Hindernis erfolgt. Das ist deshalb wichtig, weil Sterilisationsmedium über Sterilfilter, Weichverpackungen oder lange Eintrittswege geleitet werden müssen, um medizinische Instrumente zu sterilisieren.

Dabei können längere Kanäle/Leitungen unter oder vor der Teststreifenkammer mit einer innenliegenden Teststreifenhalterung vorhanden sein. Die Anordnung der Leitungen kann auch ein schneckenartig angeordneter Schlauch oder Röhre sein, damit der Dampf dieses Hindernis erst durchströmen muss, um den Teststreifenrelevanten Kontrollbereich (z. B. die chemische Reaktionsfläche) zu erreichen. Damit simuliert die Kontrollvorrichtung die Durchströmbarkeit z. B. von längeren Instrumentenkanälen, wie sie bei Endoskopen oder MIC-Instrumenten vorkommen. Wesentlich dabei ist, dass das Sterilisationsmedium erst diese Hindernisse überwinden muss, um den Kontrollbereich zu erreichen. Das Hindernis kann dabei so aufgebaut und ausgestaltet sein, wie das zu sterilisierende Sterilgut und seine Inneren Kanäle. Besonders diese Ausgestaltung verbessert die Sicherheit des Sterilisationsprozesses für schwer zu sterilisierende Sterilgüter bei dem insbesondere der Temperaturaustausch im inneren eines medizinischen Instrumentes erschwert ist. Es versteht sich dabei von selbst, dass auch die eingesetzten Temperatursensoren ebenfalls in Verbindung mit derartigen Hindernissen stehen, um den Wirkeinfluss z. B. von Dampf beeinflussen zu können. Bekannte Kontrollvorrichtungen zur Analyse der dampfbasierten Sterilisierung besitzen derartige Vorrichtung nicht.

Weiter kann aber auch eine Kontrollvorrichtung vorgesehen sein, bei der im Deckel ein Sensor eingearbeitet ist und der sensoraktive Teil in dem Innenraum des Sterilisationsbehälters angeordnet ist. Der Temperatursensor kann über ein Kabel in unmittelbarer Umgebung des Sterilgutes angeordnet werden. Derartige Ausgestaltung ist bei der Validierung von medizinischen Sterilgut in einem Sterilisationsprozess von Vorteil.

Ziel der Neuerung ist, die unmittelbar einwirkende Sterilisationstemperatur eines Sterilisationsmediums im Inneren eines Sterilisationsbehälters oder einer Sterilisationsverpackung zu bestimmen. Diese kann je nach Lage des Behälters, seiner Größe und dem eingelagerten Sterilisationsgutes abweichen. Dadurch wird mehr Sicherheit für das zu sterilisierende Produkt erreicht, als wenn nur außen am Sterilisationsbehälter ein Kontrollstreifen angebracht ist, da diese Fläche immer gut vom Sterilisationsmedium wie Dampf erreichbar ist.

Zusammenfassend verbessert die Neuerung die Sicherheit bei der medizinischen Instrumentenaufbereitung, insbesondere Abläufe in bekannten Dampfsterilisatoren und eignet sich zur Validierung von Sterilisationsprozessen. Dabei kann auch eine strukturierte Edelstahlfläche als Kontrollfläche zur Bestimmung von Silikatablagerungen zum Einsatz kommen.

### Einsatzgebiete und Ausführungsformen

Das bevorzugte Einsatzgebiet der Neuerung ist die nachgelagerte Kontrolle von Sterilisationsprozessen von medizinischen Sterilgütern aller Art und Ausgestaltung, die beispielsweise in Siebkörben gelagert und die in Sterilbehältern eingesetzt werden. Hierzu gehören kleinlumige Produkte wie Augeninstrumente, HNO-Sauger, Saver-Blads, Dentalbohrer, Sonospitzen, aber auch großlumige Produkte wie MIC-Instrumente oder ganze chirurgische Instrumentensets. Das Einsatzgebiet ist jedoch nicht ausschließlich auf medizinische Produkte beschränkt.

Als eine sinnvolle Ausgestaltung kann vorgesehen sein, dass zumindest im seitlichen Randbereich eines Sterilbehälterdeckels sich zumindest eine Kontrolleinheit befindet. Der mittlere Bereich des Deckels ist in der Regel für den Sterilfilter vorgesehen. Die Kontrolleinheit besteht hier aus einer Edelstahlfläche mit seitlich angebrachten Schienen mit innenliegenden Streifenfedern zur Festlegung der Teststreifen. Die Edelstahlfläche ist dabei wenige mm unterhalb des Sichtfensters angebracht. Das Sichtfenster und die Teststreifenhalterung kann dabei eine Einheit und eine austauschbare Vorrichtung sein, welche über Schrauben in Verbindung mit einer Dichtung in der Deckelfläche eingelassen ist. In dieser Ausgestaltung ist der Kontrollraum zumindest von zwei Seiten für das Sterilisationsmedium (z. B. Dampf) zugänglich.

In einer weiterführenden Ausgestaltung ist der Aufbau ähnlich wie bei einer frei zugänglichen Teststreifenhalterung, jedoch die Seiten sind geschlossen und besitzen an jeder Seite ein kleines Einleitloch oder einen einleitenden Kanal. Zur Entnahme des Kontrollstreifens wird vorzugsweise die Kontrollstreifenhalterung nach Öffnen einer Verschlussvorrichtung aufgeklappt. Diese Kontrollstreifenhalterung kann nach unten hin eine schneckenförmige Kanalzuführung oder eine Lochabdeckung als Hindernis aufweisen. Durch die seitlichen Öffnungen wird die Einströmmenge genau definiert und ist auf das enthaltende Sterilgut abstimmbar. Dabei entspricht vorzugsweise die Einströmöffnung die Größe des kleinsten vorhandenen Instrumentenkanals. Länge und zeitliche Durchflussmenge können dabei über einen schneckengeführten Kanal beeinflussbar sein. Die hier eingebrachten Kontrollstreifen, können aber auch eine abschmelzbare Schicht aufweisen unter der eine weitere Schicht mit Lebendkeimen (vorzugsweise Sporen) liegt. Die abschmelzbare Schicht ist dabei eine Schutzschicht für die darunterliegende Schicht. Erst nach Abschmelzen einer Deckschicht kann z. B. Dampf seine Sterilisationswirkung voll entfalten. Ist die Sperrschichtdicke entsprechend stark und schmilzt diese erst bei 130 - 134 °C innerhalb von Sekunden, so kann hiernach erst der Dampf sterilisierend bei 134 °C wirken.

Die Neuerung kann auch unabhängig von einer Weichverpackung oder einen Sterilbehälter eingesetzt werden. Hierbei wird eine öffenbare Kontrollkammer mit einem Sichtfenster eingesetzt. Vorzugsweise besteht die Kammer aus einem isolierenden Material, um nur die Einwirkung des Sterilisationsmediums zu bestimmen. Die Kammer funktioniert dabei wie eine Isolationskammer mit definierten Zuleitungen oder Hindernissen. Unter dem Sichtfenster befindet sich eine Kontrollstreifenhalterung, in der ein Teststreifen leicht entnehmbar angeordnet ist. Um einer für das Sterilisationsmedium überwindbare Barriere/Hindernis herzustellen, gehen von der Kammer z. B. längere Leitungen ab. Alternativ kann die Kammer auch Filter enthalten, ähnlich wie die in Sterilbehälter eingesetzt, besitzen. Geeignete Filter bestehen dabei aus Teflon oder mehreren Papierschichten, die zuerst vom Dampf überwunden werden müssen, um eine Sterilisation zu erreichen.

In einer anderen Ausgestaltung kommt kein Sichtfenster, sondern eine Sensoreinheit als Kontrollvorrichtung zum Einsatz, welche von außen kommend ausgelesen werden kann. Die Vorrichtung besteht dabei aus einen nach innen gerichteten Temperatur- und/oder Drucksensor welcher die ermittelten Daten an einem integrierten Datenspeicher weiterleitet. Die Vorrichtung wird durch eine Batterieeinheit mit Strom versorgt und ist autark. Auch hier kann vorgesehen sein, dass der Sensor in Verbindung mit einem Schlauch steht. Also der Dampft strömt dabei zuerst durch den Schlauch oder das Rohr und erreicht erst nach einer zurückgelegten Strecke den Temperatursensor. Auch hier wirkt der Schlauch als Ein- oder Anströmhindernis für den Dampft. Ziel ist den Sterilisationsprozess so auszuführen, dass auch schwierig zu sterilisierende Medizinprodukte und Instrumentendesigns sicher sterilisiert werden.

Eine weitere technische Ausgestaltung nutzt als Sichtfenster einen Lichtleiter oder ein auslesbares Kamerabild einer Kamera, der die Kontrollvorrichtung bildet oder Teil dieser ist. Diese Vorrichtung besitzt einen Anschlussadapter, der mit einem Lichtleiter verbunden ist. Der Lichtleiter weist an der vorderen Spitze eine kleine temperatursensitive Fläche auf, um hier eine Farbänderung über eingebrachtes Licht zu ermitteln. Diese Art der Änderung kann über ein Softwareprogramm über eine Pixel-Änderung analysiert werden. Vorteil dieser Vorrichtung ist, dass der Lichtleiter in der unmittelbaren Umgebung des Sterilgutes platzierbar ist. Desweiteren ist die Abdichtung am Behälterdeckel nach außen einfach zu realisieren.

Weitere Vorteile der Erfindung ergeben sich aus der Wahl der kombinierten Bauteile und Materialien sowie aus der Beschreibung und den beigefügten Zeichnungen.
Es versteht sich, dass die vorstehenden genannten und die nachstehenden noch zu erläuternden Merkmale nicht nur in der jeweils angegebenen Kombination, sondern zum Teil auch in anderen Kombinationen oder in Alleinstellungen verwendbar sind. Ausführungsbeispiele der Erfindung sind in den Zeichnungen dargestellt und werden in den nachfolgenden Beschreibungen näher erläutert.

Es zeigen:
- Fig. 1: Unteransicht von Sterilbehälterdeckel mit einer Kontrollvorrichtung.
- Fig. 2: Kontrollsymbole für Kontroll-Etiketten.
- Fig. 3: Schnittdarstellung einer Kontrollvorrichtung mit einer einfachen Kontrollstreifenhalterung.
- Fig. 4: Schnittdarstellung einer Kontrollvorrichtung für Kontrollstreifen in einem Sterilbehälterdeckel.
- Fig. 5: Schnittdarstellung eine Kontrollvorrichtung in Verbindung mit einem Lichtleiter in einem Sterilbehälterdeckel.
- Fig. 6: Transparente Vorrichtung mit einer Kontrollstreifenhalterung.
- Fig. 7: Darstellung ein Kontrollstreifens und sein Schichtaufbau.

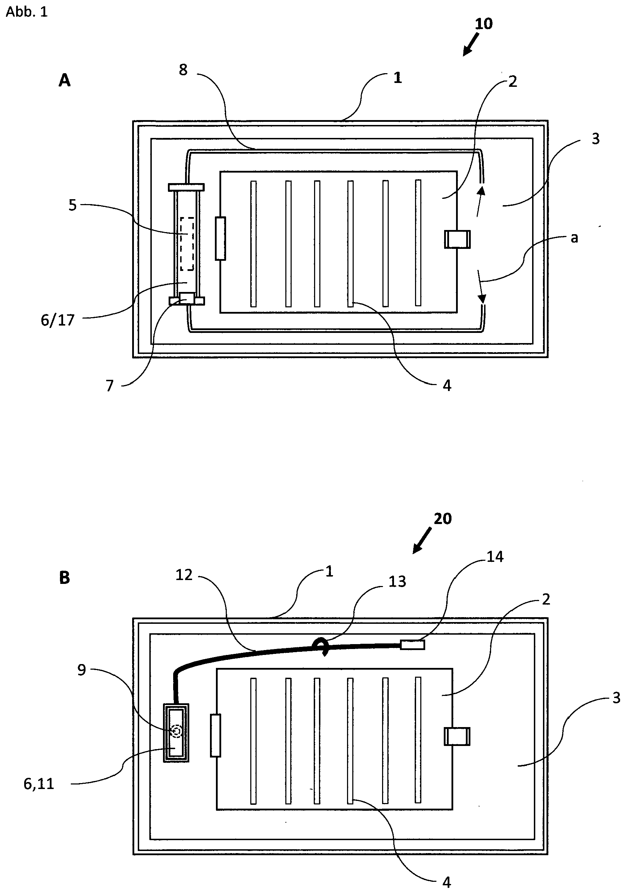

Figur 1 zeigt eine Unteransicht eines Sterilbehälterdeckels mit einer Kontrollvorrichtung. Die Abbildung **10** zeigt in **A** die Unterseite **3** eines Sterilbehälterdeckels **1** mit einer Dichtung (nicht dargestellt) und einer Aufnahmevorrichtung **2** (Sterilfilterabdeckung) für Sterilfilter mit Durchbrüche **4.** An der Deckelunterseite **3** befinden sich eine Zuführleitung (Rohrleitung, Schlauch, Kanal) **8** für die Durchleitung von Dampf (dargestellt durch **Pfeil a)** sowie ein transparentes Fenster **5** bzw. eine Luppe zur Leseunterstützung. Die Zuführleitung **8** kann dabei fest oder lösbar mit dem Deckel verbunden sein und eine gerade oder ähnlich einer Schnecke positionierten Leitung aufweisen. Unter dem transparentem Fenster **5** befindet sich eine Kontrollvorrichtung **6,** in der ein Kontrollstreifen oder ein Kontrolletikett in einem Kammerraum **17** eingebracht werden kann. Die Kontrollvorrichtung **6** besitzt zum Einbringen der Kontrollkörper eine Clipvorrichtung **7.** Die Kontrollvorrichtung **6** wird durch z. B. eine Dichtung an den Unterseite **3** des Sterilbehälterdeckels angepresst. Über die Rohrleitung wird in die Kontrollvorrichtung der Dampf eingeleitet. Die Zuführleitung **8** ist so gewählt, dass der Dampf wie bei einem Hohlraumprodukt einen längeren Weg zurücklegen muss, um eine sichere Hohlinnenraum Sterilisierung zu simulieren. Alternativ, kann auf eine Zuführleitung für Dampf verzichtet werden, insbesondere dann, wenn keine Hohlraumprodukte sterilisiert werden sollen. Die Darstellung **B,** zeigt den gleichen Sterilbehälterdeckel **1** wie in Darstellung **A,** besitzt hier eine Kontrollvorrichtung **6** mit einem Datenspeicher **11,** der von außen über einen Anschluss **9** oder hier eine Kamera aufweist, die über einen Adapter auslesbar ist. Der Datenspeicher (mit Elektronik und Batterie) ist mit einer Sensorleitung **12** verbunden, um über einen vorderen Temperatursensor **14** die Temperatur zu messen. Die Daten werden im Datenspeicher **11** mit einem zeitlichen Ablauf gespeichert und können über den Anschluss ausgelesen werden. Alternativ kann die Auslesung auch kontaktlos erfolgen. Alternativ kann der Sensor sich in oder an einer Dampf- durchleitbaren Schlauch- oder Rohrleitung befinden oder mit dieser verbunden sein, um die Sterilisierbarkeit von Kanälen mit Dampf in einem Hohlraumprodukt zu simulieren. Der an der Deckelunterseite **3** anliegende Sensorkabel **12** am Sterilbehälterdeckel **1** ist hier mit einer Sensorkabelhalterung **13** fixiert und kann auch in dem Sterilbehälterraum eingebracht werden, um insbesondere die Sterilisationstemperatur in unmittelbarer Nähe eines zu sterilisierenden Sterilgutes zu prüfen.

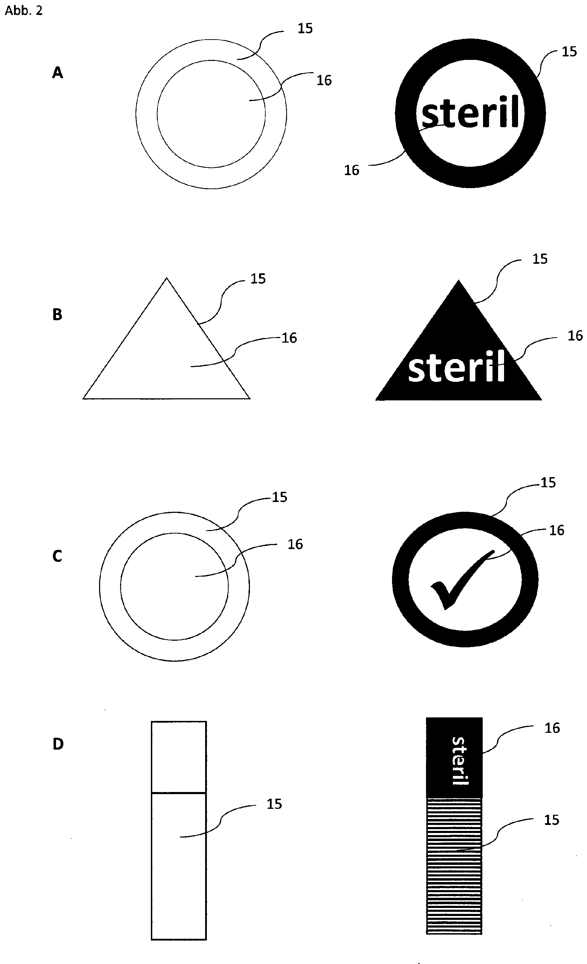

Figur 2 zeigt Kontrollsymbole für Kontroll-Etiketten. Die Darstellung **A** zeigt ein Kreis-Symbol **15** mit einem kaum lesbaren Wort **16** (hier das Wort "steril"). Die Symbolfarbe ist hier so gewählt, dass sowohl das Kreis-Symbol **15** und das Wort **16** ganz deutlich bei einem erfolgreichen Sterilisationsprozess vorzugsweise in einer anderen deutlich sichtbaren Farbe erscheint. Alternativ kann hier sich auch nur der zentrale Kreiskern farblich ändern und damit das Wort sichtbar machen. In B ist ebenfalls ein Kontroll-Etikett darstellt als Dreieck-Symbol **15** dargestellt. Das in dem Symbol innenliegenden Wort ist dabei kaum oder nicht lesbar. Erst nach einer erfolgreichen Sterilisation erscheint das Wort **16** "steril" auf Grund eines sich anders farbigen oder dunkleres Hintergrundes. Jetzt ist auch das Wort "steril" gut lesbar. Die Darstellung **C** wiederum zeigt wie in **A** ein Kreis-Symbol **15** in einer schwachen Anfärbung (ein Zeichen für nicht steril) und einem international bekannten "okay" **16** Zeichen, welches kaum lesbar oder überdeckt sein kann. Diese Darstellung zeigt nach außen an, dass etwas nicht okay ist. Nach dem Sterilisieren erscheint das "okay" Symbol sowie der umgebende Kreis und gibt an, dass alles "okay" ist, also steril ist. Die Darstellung **D** zeigt einen Kontroll-Streifen, der in der unsterilen Fassung keinen oder einen kaum sichtbaren Hintergrund **15** aufweist. Erst nach einer korrekt erfolgten Sterilisation wird hier z. B. ein Barcode und ein Wort "steril" **16** deutlich sichtbar und der Barcode ist jetzt erst auslesbar. Die oberen Darstellungen **A, B, C** und **D** visualisieren somit nicht nur einen Sterilisationsprozess durch eine Farbänderung, sondern gleichzeitig erhöhen die Symbole **15** und die Wörter oder Zeichen **16** den Informationsgehalt. Dabei kann anstelle einer Farbumwandlung eine Farbfreisetzung stattfinden und nach Entfernung dieser Schicht können die beschriebenen Symbole sichtbar werden. Wichtig ist dann, dass das Schmelzen der Deck- oder Sperrschicht kontrolliert erfolgt. Es kann dabei das Ziel sein, dass z. B. eine 5-minütige einwirkende Sterilisationszeit eingehalten wird.

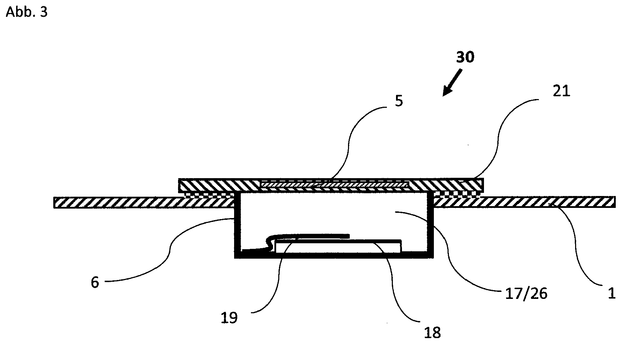

Figur 3 zeigt in Schnittdarstellung eine Kontrollvorrichtung mit einer einfachen Kontrollstreifenhalterung. Die Darstellung **30** ist dabei vorzugsweise austauschbar in einem Sterilbehälterdeckel **1** eingebracht. Alternativ kann die Kontrollvorrichtung als eingeständige Einheit eine Batterie, einen Datenspeicher und Elektronik enthalten und ermittelte Pixel- oder Kontrolldaten nach außen senden. Es befindet sich ein transparentes Sichtfenster **5** bzw. eine Luppe in einer Abdeckplatte **21** und hierrunter befindet sich beabstandet ein Kontrollstreifen mit einer z. B. Temperaturmarker-Oberfläche **18** (Kontrollfläche), der über eine Haltefeder **19** fixiert ist. Hier kann das Sterilisationsmedium den offenen Raum der Kontrollstreifenhalterung **26** zumindest über die Seitenflächen den Kontrollstreifen leicht erreichen. Von Vorteil ist es, wenn die Fensterfläche eine Lupe ist, um die Kontrollfläche **18** auf dem Teststreifen besser beobachten zu können.

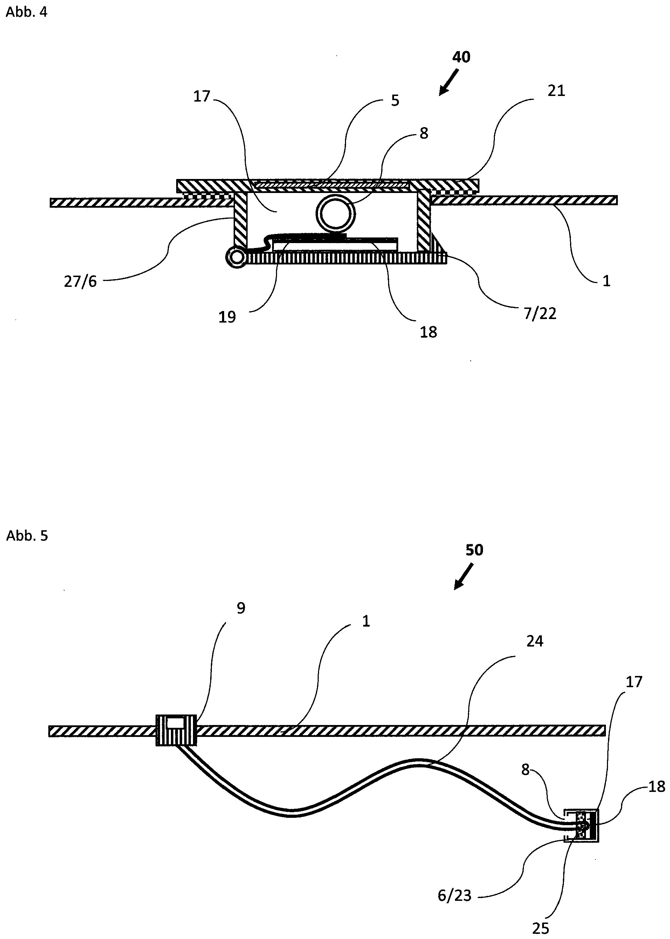

Figur 4 zeigt in Schnittdarstellung eine Kontrollvorrichtung für Kontrollstreifen in einem Sterilbehälterdeckel **1.** Die Darstellung **40** ist eine Kontrollvorrichtung **6** in Ausgestaltung einer Kontrollkammer **27** die öffenbar ist. Die Kammer **27** ist eine montierbare Einheit mit einer Abdeckplatte **21,** welche mit in einer Sterilbehälterdeckel **1** fixiert (z. B. verschraubt oder verklebt oder ähnlich) ist. Alternativ kann die gezeigte Kontrollvorrichtung **6** in einer ähnlichen Ausgestaltung alleine in einer Weichverpackung oder auch unabhängig von einer Umverpackung eingesetzt sein. Das kann dann sinnvoll sein, wenn die Sterilisation in einer Sterilisationskammer an unterschiedlichen Stellen, untersucht werden soll. Bei der Darstellung **40** wird das Sterilisationsmedium z. B. Dampf über eine Rohrleitungsöffnung **8** in den Innenraum **17** der Sterilisationskammer **27** eingeleitet. Alternativ kann hier auch ein Schlitz oder eine Öffnung **8** vorhanden sein. Um den Kontrollstreifen mit der Kontrollfläche **18** in der Vorrichtung schnell wechseln zu können ist dieser über einer Feder **19** am Vorrichtungsdeckel **22** der Kontrolleinheit leicht entnehmbar fixiert. Der Deckel **22** der Kontrollkammer **27,** vorzugsweise als Kontrollstreifenhalterung ausgebildet, wird z. B. mit einem Clipverschluss **7** oder einer Verschraubung sicher geschlossen. Alternativ kann hier der Dampf über eine Öffnung in den Vorrichtungsraum **17** einströmen oder der Kontrollhalterungsdeckel selber oder besitzt an der Kammerseite eine Hindernisstrecke in Form einer Schnecke mit zumindest einer Eintrittsöffnung in die Kontrollkammer **27.** Die Abdeckplatte **21** besitzt ein transparentes Fenster **5** mit oder ohne Lupenfunktion. Über das Fenster **5** kann man den beabstandeten Kontrollstreifen sehen und deren Farbänderung nach der Sterilisierung beobachten. Technisch kann vorgesehen sein, dass die Kontrolleinheit **40** als ganze Einheit auswechselbar ist. Ferner kann vorgesehen sein, dass mehrere Kontrolleinheiten hintereinander angeordnet und mit einem Schlauch miteinander verbunden sind, um eine Doppelbestimmung zu erzielen. Der Kontrollstreifen selber kann aus einem Filtermaterial bestehen, um abgeschmolzene Materialien aufzunehmen.

Figur 5 zeigt in Schnittdarstellung eine Kontrollvorrichtung in Verbindung eines Lichtleiters in einem Sterilbehälterdeckel **1.** In dieser Darstellung **50** ist ebenfalls eine Kontrollvorrichtung beschrieben. Hier ist ein Lichtleiter **24** mit einem Anschlussadapter **9** mit einem Sterilbehälterdeckel **1** vorzugsweise verschraubt. Der Lichtleiter **24** kann dabei frei im Sterilbehälterraum oder über eine Fixierung am Deckel festgelegt sein. Am Ende des Lichtleiters **24** befindet sich eine auswechselbare Kappe **23,** die am Boden eine Kontrollfläche **18** aufweist. Die Kappe mit der innenliegenden temperatursensitiven Kontrollfläche **18** und einem Kappenraum **17** ist leicht auszutauschen und bildet zusammen mit dem Lichtleiter **24** die Kontrollvorrichtung. Vorteil dieser Kontrollvorrichtung ist, dass die Farbänderung auf kleinstem Raum dedektierbar ist und die Auswertung über ein Pixel-Analyse erfolgen kann. Letzteres kann von Vorteil sein, wenn man eine Standardisierung anstrebt, um insbesondere Sterilisationsdaten weltweit miteinander austauschen und vergleichen zu können. Die Kappe **23** kann dabei so aufgebaut sein, dass der Dampf erst über die Öffnungen 8 und hiernach über ein Hindernis oder Barriere **25,** welche der Kontrollfläche vorgelagert ist, geleitet werden muss, um zur Kontrollfläche **18** zu gelangen. Das vorgelagerte Hindernis kann dabei ein Filtermaterial sein, dass das abgeschmolzene Material (Deckschicht) aufnimmt. Damit entspricht die Vorrichtung einer Bowie-Dick Testvorrichtung. Alternativ kann der Lichtleiter sich in einer längeren Schlauchleitung oder Rohrleitung befinden und diese nach außen hin über ein Filterelement an den Enden verschlossen ist.

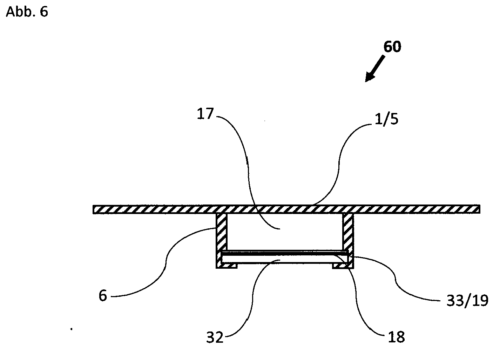

Figur 6 zeigt eine transparente Vorrichtung mit einer Kontrollstreifenhalterung. Die transparente Vorrichtung ist hier als Deckel **60** dargestellt und besteht vorzugsweise aus einem transparenten Kunststoff. Alternativ ist diese Vorrichtung **60** in ähnlicher Form auch für sich in Weichverpackungen nutzbar. Der Deckel ist so ausgebildet, dass hier eine Kontrollstreifenhalterung **33** aus dem Deckelmaterial herausgearbeitet ist oder aus diesem Material geformt wurde. Hier wird ein Kontrollsteifen **32** vorzugsweise seitlich eingebracht. Dabei kann vorgesehen sein, dass beim Einbringen des Kontrollstreifens dieser mechanisch beschädigt wird, um eine zweifache Nutzung zu verhindern. Die Halterung **33** besteht vorzugsweise aus dem gleichen oder ähnlichen Material wie der Deckel und könnte durch z. B. Spritzguß oder im 3 D-Verfahren hergestellt werden. Zur besseren Fixierung der Kontrollstreifen besitzt die Halterung in der Haltenute ausgeformte seitliche Halteteile oder eine Haltefeder **19,** um den Kontrollstreifen vorzugsweise kraft- oder formschlüssig aufzunehmen. Auch diese Vorrichtung **60** kann seitliche Hindernisse aufweisen oder mit diesen verbunden sein.

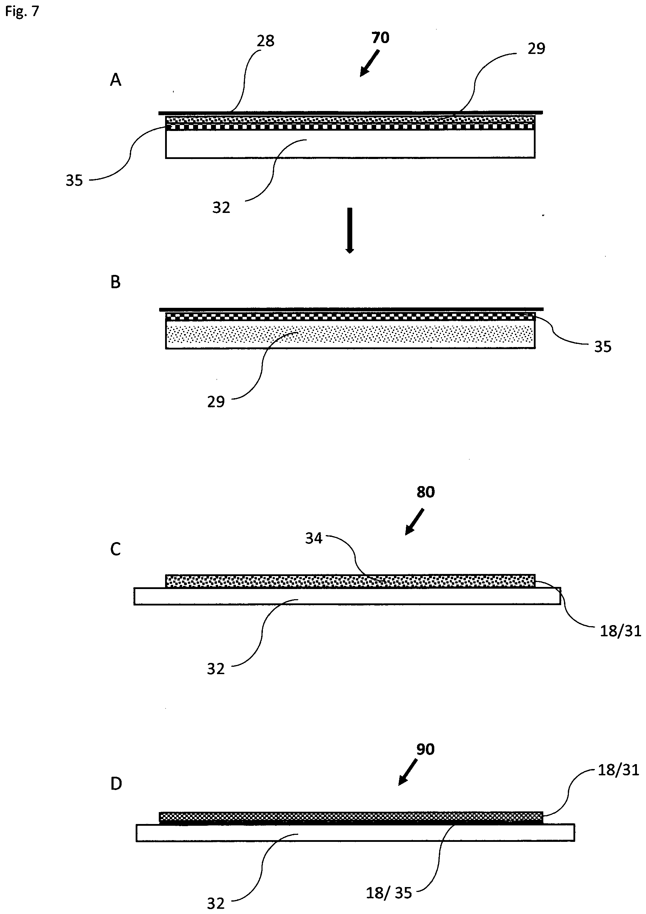

Figur 7 zeigt eine Darstellung eines Kontrollstreifen mit Schichtaufbau. Der Kontrollstreifen **70** in Darstellung **A** besteht aus unterschiedlichen Schichten. Die erste Schicht ist eine transparente Schutzschicht **28.** Diese Schicht kann alternativ auch nicht vorhanden sein, wenn das Produkt in Reinigungsmaschinen eingesetzt werden soll. Die zweite Schicht **29** bildet ein schmelzbares Material. Unter dieser Schicht befindet sich eine temperatursensitive Indikatorschicht **35,** welche auch Metallkristalle zur Temperaturanzeige enthalten kann. Diese Schicht reagiert bei einer definierten Temperatur mit einer Farbänderung. Die obigen Schichten **28, 29** und **35** sind auf ein Trägermaterial **32** wie z. B. Papier oder auf einem lösungsstabilen Kunststoffträger mit oder ohne einer Filterpapierlage aufgetragen. Von Vorteil sind saugfähige Trägermaterialien **32,** um das geschmolzene Material **29** aufzunehmen. Die Darstellung **B** verdeutlicht, dass das geschmolzene Material der Schicht **29** bei einer eingestellten Schmelztemperatur in das Trägermaterial **32,** beispielsweise über Kanäle fliest oder durch dieses Material **32** wandert bzw. diffundiert. Vorzugsweise kann die Schicht **35** Testkeime oder anderes Biomaterial enthalten. Nach einer erfolgten Temperatureinwirkung wird die Schutzschicht entfernt und der Kontrollstreifen (auch in runder Form) mit einer Agarschicht in einer Petrischale in Kontakt gebracht. Nach einem definierten Kontakt mit dem Agar wird die Petrischale für mehrere Tage in einer Inkubatorkammer eingebracht, um das Bakterienwachstum zu beobachten. Darstellung **C** zeigt einen Kontrollstreifen **80** mit einer Wachstumsschicht **31** für Keime die als Fläche auf dem Kontrollstreifen **32** aufgebracht ist. In dieser Schicht können Temperaturindikatoren **34** eingebracht sein, um bei Erreichen der Sterilisationstemperatur einen Farbumschlag durchzuführen. Derartige Teststreifen werden bereits steril, vorzugsweise mit einer Schutzschicht, eingesetzt. Darstellung **D** zeigt einen Kontrollstreifen **90,** wobei hier über einer Indikatorschicht **35** ein fester Nährboden **31** (Wachstumsschicht oder - fläche) liegt. Durch die Dicke dieser Wachstumsschicht **31** wird der eingebrachte Dampf oder das Sterilisationsmediums mehr oder weniger verzögert wirken und so sichergestellt, dass das Sterilisationsmedium auch unter diesen Bedingungen wirkt. Die hier gezeigten Teststreifen A, B, C und D können über eine Halterung in einem Siebkorb oder in einem Sterilcontainer eingebracht werden.

### Zeichenerklärung

- 1.: Sterilbehälterdeckel oder -wandung
- 2.: Sterilfilterabdeckung oder Aufnahmevorrichtung für Sterilfilter
- 3.: Innenfläche des Sterilbehälterdeckels
- 4.: Sterilfilteröffnungen im Deckel
- 5.: Transparentes Fenster, Luppe oder transparenter Deckel
- 6.: Kontrollvorrichtung
- 7.: Verschluss an der Kontrolleinheit
- 8.: Zuführleitung- oder -kanalöffnung (z. B. eines Schlauches oder Rohrs) oder nur eine Öffnung
- 9.: Adapter oder Kamera mit einen Ausleseadapter
- 11.: Datenspeicher mit Elektronik und Batterie
- 12.: Sensorkabel oder -leitung
- 13.: Halterung für ein Sensorkabel
- 14.: Temperatursensor
- 15.: Symbol oder Teststreifen mit einer vorzugsweise temperatursensitiven Farbgebung
- 16.: Wort oder Zeichen
- 17.: Innenraum einer Kammer oder Kappe einer Kontrollvorrichtung
- 18.: Kontroll- bzw. Markerfläche eines Test- oder Kontrollstreifens oder einer Kappe
- 19.: Halterung oder Haltefeder für einen Kontrollstreifen
- 21.: Abdeckplatte der Kontrolleinheit
- 22.: öffenbarer Deckel der Kontrolleinheit (Kontrollhalterungsdeckel)
- 23.: Kappe als Kontrollflächenhalterung
- 24.: Lichtleiter
- 25.: Hindernis, Sterilbarriere oder Sterilfilter
- 26.: offener Raum einer Kontrollstreifenhalterung
- 27.: Kontrollkammer
- 28.: transparente Schutzschicht
- 29.: schmelzbare Schicht
- 31.: Wachstumsschicht als feste Nährstoffschicht die biologisches Material und/oder temperatursensitive Stoffe enthält oder aus diesem besteht
- 32.: Trägermaterial oder Kontrollstreifen
- 33.: Kontrollstreifenhalterung als Deckelbestandteil
- 34.: Farbindikatoren oder temperatursensitive Stoffe
- 35.: Indikatorschicht

## Patentansprüche

1. Sterilisationsvorrichtung mit einer Kontrollvorrichtung für Sterilisationsprodukte, die in Sterilisationsgeräten zum Einsatz kommt, wobei die Sterilisationsprodukte in einem Sterilbehälter oder einer transparenten Weichverpackung als Sterilisationsverpackung eingebracht sind, **dadurch gekennzeichnet, dass** eine Kontrollvorrichtung (6, 23) zumindest eine Kontrollfläche (18, 31) im inneren einer Sterilisationsverpackung so anordnet, dass das Sterilisationsmedium bei der Sterilisation zumindest die Kontrollfläche in der Kontrollvorrichtung über eine offene Seite (26), eine Öffnung oder einen Kanal (8) und/oder über ein Hindernis (25, 31) erreicht, um eine sichtbare Flächenänderung der Kontrollfläche zu bewirken und diese ohne Öffnen der Sterilisationsverpackung zumindest außerhalb des Sterilisationsgerätes visuell oder mit optischen Methoden auszulesen oder zu analysieren.

2. Sterilisationsvorrichtung nach Anspruch 1, **dadurch gekennzeichnet, dass** die Kontrollvorrichtung (6, 23) ein fester Bestandteil eines Sterilbehälterdeckels (1), eine auswechselbare oder eine einlegbare Einheit oder ein Bestandteil eines Siebkorb ist, deren Kontrollfläche (18, 31) über ein transparentes Fenster (5) in einer starren Sterilisationsverpackung oder eine Folie einer Weichverpackung oder über eine integrierte Lupe, einen Lichtleiter (24) und/oder ein Kameramodul (9) optisch erfassbar ist, um zumindest eine visuelle oder optische Analyse der zugänglichen Kontrollfläche zu ermöglichen.

3. Sterilisationsvorrichtung nach Anspruch 1 und 2, **dadurch gekennzeichnet, dass** die Kontrollfläche (18, 31) in einer Kontrollstreifenhalterung (19, 33) zumindest form- oder kraftschlüssig fixierbar ist oder sich diese in einer Kappe (23) befindet und hierüber ein Kontrollkammerraum (17) angeordnet ist, der seitlich offen (26) oder zum Teil eine geschlossene Kontrollkammer (27) ist und/oder zumindest ein Hindernis (8, 25, 31) für Sterilisationsmedium aufweist.

4. Sterilisationsvorrichtung nach Anspruch 1 bis 3, **dadurch gekennzeichnet, dass** die Kontrollvorrichtung (6, 23) Sterilisationsmedien über eine Filterschnecke heranführt oder das Sterilisationsmedium muss erst ein Filtermaterial (25) in einer Kappe (23) oder eine Wachstumsschicht (31) auf einem Trägermaterial (32) überwinden bevor es die Kontrollfläche (18) erreicht.

5. Sterilisationsvorrichtung nach Anspruch 1 bis 4, **dadurch gekennzeichnet, dass** die Kontrollvorrichtung (6, 23) zumindest eine Kontrollfläche (18, 31) aufweist, welche eine abschmelzbare Schicht (29) und/oder eine symbolzeigende Indikatorschicht oder Symbolfläche (15) oder eine Wachstumsschicht (31) für Keime mit einem Indikator (34) besitzt und die Kontrollvorrichtung (6) außer- und innerhalb einer Sterilverpackung nutzbar ist.

6. Sterilisationsvorrichtung nach Anspruch 1 bis 5, **dadurch gekennzeichnet, dass** Sterilisationsmedien zumindest über eine Zuführleitung (8), einen Spalt und/oder über Filtermaterialien zugeführt wird und dadurch eine Zeitverzögerung der Einwirkzeit und/oder der Einwirkkonzentration des Sterilisationsmediums erreicht wird.

7. Sterilisationsvorrichtung nach Anspruch 1 bis 6, **dadurch gekennzeichnet, dass** die Kontrollvorrichtung (6, 23) aus einem Temperatursensor (14), einer Batterie und einen Datenspeicher (11) besteht, um innerhalb einer Sterilverpackung die Daten zumindest über einen Adapter (9) oder kontaktlos auszulesen.

8. Sterilisationsvorrichtung nach Anspruch 1 bis 7, **dadurch gekennzeichnet, dass** eine optische Analyse oder das Auslesen der Daten bereits im Sterilisationsgerät über ein Kabel oder über Funkt erfolgt.

9. Kontrollvorrichtung (6, 23) in einer Sterilisationsvorrichtung nach einem der obigen Ansprüche, **dadurch gekennzeichnet, dass** diese ein transparenter Deckel, ein Sichtfenster (5) oder eine Luppe in einer Sterilisationsverpackungswandung, ein Lichtleiter oder ein Siebkorb ist und eine Kontrollhalterung aufweist, um zumindest die eingewirkte Sterilisationstemperatur auf der Kontrollfläche (18, 31) zumindest in Form einer Farb- oder Symboländerung (15) und/oder einer Koloniebildung auf oder in einer Wachstumsschicht (31) im inneren einer Sterilisationsverpackung zu ermitteln oder bestimmen zu können.

10. Kontrollvorrichtung nach Anspruch 9, **dadurch gekennzeichnet, dass** diese einen schneckenartigen oder längeren Schlauch oder Röhre besitzt, um hierüber Dampf in eine Kontrollkammer (17) einzuleiten, um die Sterilisierung von längeren Instrumentenkanälen zu prüfen, ohne die Sterilverpackung öffnen zu müssen oder ein Kontrollhalterungsdeckel (22) oder eine Kammerseite besitzt, welche eine Hindernisstrecke in Form einer Schnecke mit einer Eintrittsöffnung in die Kammer aufweist.

11. Kontrollfläche in einer Kontrollvorrichtung zur Prüfung der Sterilisation nach Anspruch 9, **dadurch gekennzeichnet, dass** die Kontrollfläche (18, 31) als Teststreifens (70, 80, 90) in der Sterilisationsvorrichtung (6) eingebracht ist und als Kontrollfläche zumindest eine Wachstumsschicht (31) für Keime aufweist, wobei diese über- oder nebeneinander einer Indikatorschicht (35) angeordnet ist oder die Kontrollfläche (18, 31) aus einer Wachstumsschicht für Keime besteht, die zumindest Farbindikatoren enthält.

12. Kontrollvorrichtung nach Anspruch 9 bis 11, **dadurch gekennzeichnet, dass** der Sterilisationserfolg zumindest über eine chemische Farbänderung visuell oder mit einer Kamera (9) oder Scanner über Pixeländerung oder über eine physikalisch messbare Temperaturänderung erfassbar ist.

13. Kontrollvorrichtung nach Anspruch 9 bis 12, **dadurch gekennzeichnet, dass** die Kontrollvorrichtung (6) einen physikalischen Sensor (14) endständig an einem Sensorkabel (12) aufweist und einen Datenspeicher (11) besitzt, wobei die Kontrolldaten über eine Induktionsfläche kabellos auslesbar sind.

14. Kontrollvorrichtung nach Anspruch 9 bis 13, **dadurch gekennzeichnet, dass** die eingebrachte Kontrollfläche (18) eine schmelzbare Schicht über eine Indikatorschicht (31) aufweist wobei eine temperaturgetriebene chemische Farbänderung und Abschmelzung einer oberen Schicht (29) ein Symbol (15) oder ein Symbol mit einem Zeichen oder ein Wort (16) erzeugt oder freisetzt oder die abschmelzbare Schicht (29) eine hierunterliegende Kontrollschicht mit hier enthaltenen Biomaterial freilegt wobei ein saugfähiges Material (32) die darüberliegende schmelzbare Sperr- oder Deckschicht aufnimmt oder über Kanäle in dieses Material fließen kann.

15. Kontrollvorrichtung nach Anspruch 9 bis 14, **dadurch gekennzeichnet, dass** die eingebrachte Kontrollfläche (18) eine flächige Wachstumsschicht (31) mit zumindest temperatursensitiven Stoffen enthält wobei bei der Sterilisation eine Farbänderung bewirkt wird und hiernach die sterile Wachstumsfläche zur Sterilitätskontrolle nach der Sterilisation eingesetzt ist.

16. Kontrollvorrichtung nach Anspruch 9 bis 15, **dadurch gekennzeichnet, dass** die Kontrollvorrichtung (6) mit der Kontrollfläche (18) oder nur die Kontrollfläche bei der Reinigung eingesetzt wird, wobei die aufliegende Sperr- oder Deckschicht über die Farb- oder Indikatorschicht zuerst bei der Reinigung abgewaschen und hiernach mit einer Sterilisationsvorrichtung oder die Kontrollfläche in einem Sterilisationsgerät nutzbar ist.
